Europäisches Patentamt

European Patent Office

Office européen des brevets

(11) Publication number: **0 421 099 A2**

# EUROPEAN PATENT APPLICATION

(21) Application number: 90115676.0

(22) Date of filing: 16.08.90

(51) Int. Cl.5: **C12N 15/67**, C12N 15/12, C12N 15/85

The applicant has subsequently filed a sequence listing and declared, that it includes no new matter.

(30) Priority: 25.09.89 US 411942

(43) Date of publication of application:
10.04.91 Bulletin 91/15

(84) Designated Contracting States:
AT BE CH DE DK ES FR GB GR IT LI LU NL SE

(71) Applicant: **AMERICAN CYANAMID COMPANY**
**1937 West Main Street P.O. Box 60**
**Stamford Connecticut 06904-0060(US)**

(72) Inventor: **Vitek, Michael Peter**
**213 Wyckoff Avenue**
**Waldwick, New Jersey 07463(US)**

(74) Representative: **Wächtershäuser, Günter, Dr.**
**Tal 29**
**W-8000 München 2(DE)**

(54) Methods for modulating promoter responsible for beta amyloid precursor protein expression.

(57) The present invention relates to a method for controlling the expression of the beta amyloid precursor protein, a key protein involved in Alzheimer's Disease. Further, the present invention relates to a promoter reporter system useful in studying the effects agents have on the expression of the beta amyloid precursor protein and also provides a method for treating Alzheimer's Disease by regulating activities on the promoter system.

EP 0 421 099 A2

# METHODS FOR MODULATING PROMOTER RESPONSIBLE FOR BETA AMYLOID PRECURSOR PROTEIN EXPRESSION

## BACKGROUND OF THE INVENTION

The present invention relates to a method for controlling (modulating) the expression of the beta amyloid precursor protein (APP) which is key in studying the effects of the beta amyloid peptide. This method involves using a reporter system for expressing human growth hormone (hGH) which is under the direct control of the APP promoter, specifically a promoter that has as key elements a heat shock response element (HSE) and AP-1 binding sites found in the APP promoter. Further, the present invention also relates to the specified promoter and a mechanism for measuring the regulation of the expression capabilities of the APP promoter by utilizing a reporter system linked to the expression of human growth hormone and measuring the human growth hormone expressed. Surprisingly, the present invention also relates to the fact that Interleukin-1 (human IL-1 beta) has been found to induce the present APP promoter and Interleukin-1-like compounds which act substantially in the same way on the promoter system are determined to be key in controlling the production of the amyloid precursor protein. Some such Interleukin-1-like compounds include Interleukin-6 and Tumor Necrosis Factor. Therefore, they may also be key in controlling the formation of plaques which have been found to accumulate in Alzheimer's Disease brains. Ultimately, the present invention also relates to methods for treating Alzheimer's Disease by utilizing agents which modulate the promoter system linked to the reporter system wherein human growth hormone is measured. It is obvious that such agents therefore control the expression of the amyloid precursor protein which in turn is key in the production of beta amyloid containing plaques found in Alzheimer's Disease patients' brains.

Alzheimer's Disease is the leading cause of dementia and the fourth leading cause of death in the elderly population in the USA (32). The neuropathology of Alzheimer's Disease is characterized by buildup in the brain of amyloid plaques, neurofibrillary tangles which do not contain BAP and cerebrovascular deposits consisting predominantly of a 42 amino acid peptide known as the A4 or beta amyloid peptide (BAP) (9). Recent investigation into the mechanism of BAP deposition suggests that these structures result from the overproduction and/or incorrect processing of amyloid precursor proteins (APP), particularly in the frontal cortex, hippocampus and nucleus basalis (2, 4, 13). BAP is contained within the larger precursor protein of which at least three (3) forms are known to be expressed in the brain (6, 8, 14, 22, 31). The specific form that is processed from APP to BAP is not known. The three (3) forms of APP produced through alternative splicing of the pre-mRNA differ by the insertion of a Kunitz type protease inhibitor region of 57 or 76 amino acid inserted into the smallest 695 amino acid form producing 751 and 770 amino acid forms. All three (3) forms are believed to be encoded by a single gene on the twenty first (21st) chromosome in the human genome (29). In AD brains the relative amounts of the three (3) forms may be altered resulting in the abnormal accumulation of one of the forms and its subsequent processing into the deposited BAP.

A promoter of the APP gene has been isolated and shown to contain sequences which are homologous to constitutively expressed genes and inducible genes (24). APP is found in peripheral and brain tissues (11, 29) suggesting that the constitutive promoter sequences are active in many cell types. The activity of the inducible sequences which are characteristic of AP-1 binding sites, heat shock elements and phorbal ester inducible sequences further suggests that the APP gene is activated by agents that activate the protein kinase C messenger system (1, 20) or induce the production of fos related proteins (5, 17) and/or other transcription factors involved in regulating the production of mRNA from this gene.

The single gene copy of the human APP gene, located on the twenty first (21st) chromosome does not map to the Familial Alzheimer's Disease locus (FAD) (9). The APP gene contains at least sixteen exons. Depending on splicing of the initial transcript (16), at least three forms of the protein are expressed from this gene through alternative RNA splicing of the primary transcript. Neither the pathway of protein processing of the large APP precursor to the forty-two aa BAP is known, nor is the actual form that is involved in the protein processing known.

In order to study the regulation of expression of the APP gene, the promoter region has been isolated and analyzed for activity in a cell culture reporter gene system. The promoter contains guanosine-cytosine (GC) rich regions characteristic of constitutively expressed genes including housekeeping genes vimentin (26),Nerve Growth Factor receptor (NGF) (27) and the Simian Virus type 40 (SV 40) early genes (3) but does not contain a TATA or CAAT box (These are a pattern of nucleotides found in most promoter DNAs) typical of tissue specific and other eukaryotic promoters.

In addition to the GC rich regions, this promoter contains both a heat shock responsive element (HSE) and AP-1 binding site (AP-1 is a protein complex of FOS and JUN proteins; the AP-1 binding site has a specific pattern of nucleotides) within three hundred five (305) to four hundred eighty-five (485) nucleotides of the transcription start site. These inducible promoter sequences are tested in the hGH reporter gene system for their ability to respond to IL-1.

It is found that the promoter fragments containing the HSE and AP-1 binding sites are responsive to IL-1 and increase the level of hGH production driven by these promoter fragments. The promoter fragment containing only the GC sequences without the HSE or Distal AP-1 binding sites are not responsive to IL-1 but do produce a constitutive level of hGH in the absence or presence of IL-1.

IL-1 has been shown to activate gene expression via the protein kinase C system (23), through the synthesis of the Fos-Jun proteins which act as transcription factors binding to the AP-1 binding site in the promoter DNA of other inducible genes (17). Previous studies have shown that IL-1 is able to increase production of NGF through stabilization of and increased transcription of the NGF mRNA (18). This stimulation may involve facilitation via the protein kinase C messenger system which has been shown to be involved in the stimulation of APP mRNA in human primary cell cultures. NGF also directly stimulates APP synthesis in the developing hamster brain (19) through an unknown pathway. In addition, the fos gene is shown to be directly stimulated by treatment of cells with IL-1 (17). Therefore, although not wanting to be limited by theory, it is proposed that the response to IL-1 in AB-1 (mouse neuroblastoma) cells is involved with the fos gene products and possibly other oncogene-related transcription factors. Also, since NGF production is stimulated by IL-1, it is possible that the induction seen in this study is mediated through NGF.

In order to investigate the activity of promoter regulatory sequences, an isolated APP promoter sequence is introduced into mouse neuroblastoma cells to test for activity under a variety of conditions. Surprisingly, the present invention discovered that the promoter is active in those neuroblastoma cells when the HSE and AP-1 binding sites are present and is inducible by Interleukin-1 type compounds. Therefore, the present invention suggests that this promoter is able to regulate the production of APP and response to stress inducing factors in neuronal cells, and this may be a mechanism by which BAP is overproduced leading to extra normal accumulation of BAP in Alzheimer's Disease.

## BRIEF DESCRIPTION OF THE DRAWINGS

FIGURE 1. The BamH1 - BamH1 fragment from the pUC19 subclone Containing the EcoR1 - BamH1 fragment of the lambda clone is isolated and ligated into pOGH's BamH1 site to produce pCLL EB (aka pCLL 783) and pCLL BE(aka pCLL 784) pCLL-EB is digested with HindIII or XbaI to produce pCLL-HB and pCLL-XB, respectively. All numbers in the figure refer to the transcribed sequence where +1 is the first base of the APP's open reading frame in the mRNA sequence.

FIGURE 2. DNA sequence of the promoter region cloned into pCLL-HB.

A. The DNA sequence of 485 bases of the APP promoter region.

B. Location and sequence of the potentially active regions of the APP promoter showing the consensus sequence and the location of the mismatches bases (underlined).

FIGURE 3. Human growth hormone production by AB-1 cells transiently transfected with the APP promoter constructs shown in Figure 1.

A. AB-1 cells are transfected with each of the promoter constructs and assayed after 60 hrs for production of hGH.

B. Induction of the transiently transfected AB-1 cells with 1 ng/ml Interleukin-1. Cells are assayed for hGH production after 60 hrs.

## SUMMARY OF THE INVENTION

The present invention relates to a method for modulating the expression of amyloid precursor protein wherein said method involves using a promoter for expressing said amyloid precursor protein (APP) ' containing an HSE and AP-1 binding sites. Preferably, these binding sites are within three hundred five (305) to four hundred eighty-five (485) nuclotides of the transcription start signal of a reporting system.

In addition to this objective, the present invention relates to the actual promoter wherein the key elements are the HSE and AP-1 binding sites. Further, the present invention also relates to methods for measuring the inducibility of a promoter DNA wherein this method involves inserting the promoter DNA into a plasmid which constitutes a system for expressing human growth hormone and then testing the inducibility of the promoter system with Interleukin-1 or compounds having Interleukin-1 type activity.

In addition to the above, the present invention also relates to mechanism for controlling the producing of the beta amyloid proteins by controlling the production of the amyloid precursor RNAs.

Finally, it is appreciated that the present invention also relates to the mechanism for treating Alzheimer's Disease by regulating the production of the amyloid precursor protein with Interleukin-1 agonists and antagonists or which affect this production of amyloid precursor protein in a similar manner as Interleukin-1 like compounds.

For purposes of the present invention all of the plasmids, DNA sequences and microorganisms deposited in connection with the present invention, except where specified to the contrary, are deposited in American Cyanamid Company's culture collection maintained in Princeton, New Jersey and are deposited with American Type Culture Collection in Rockford, Maryland 20952 USA, per the following:

pCLL783 in **E. coli** is ATCC 68086 deposited August 31, 1989 and mouse neuroblastoma cell line AB-1 is ATCC CRL 10211 also deposited August 31, 1989.

These and further objects of the invention will become more obvious by the detailed description of the invention provided hereinbelow.

## DETAILED DESCRIPTION OF THE INVENTION

The following are provided as examples of the invention and are not limitative thereof:

## EXAMPLE 1

## Genomic Cloning And DNA Sequencing

A chromosome twenty-one (21) library (ATCC 57743) containing EcoRI fragments is screened with a fifty-nine (59) base oligonucleotide probe homologous to the 5′ end of the APP mRNA sequence. Identified fragments are subcloned into the EcoRI site of pUC19 and sequenced using the chain termination reaction method and Sequenase\ (US Biochemicals).

## EXAMPLE 2

## Construction Of The Reporter Gene System

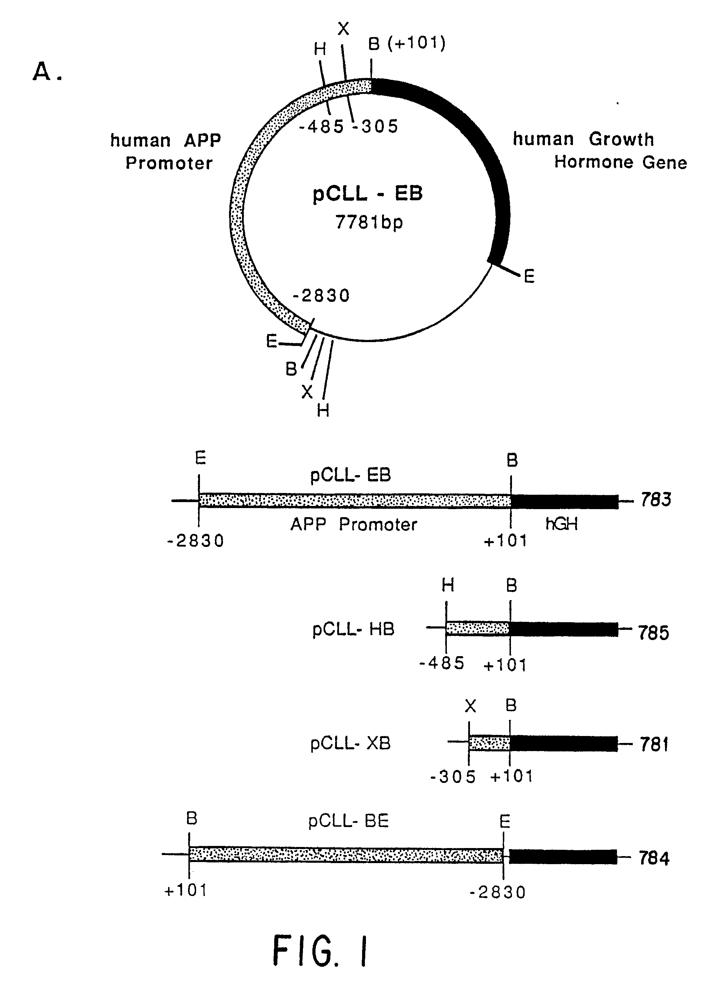

Restriction fragments from the pUC19 subclones containing the promoter region are inserted into the human growth hormone reporter gene system (Nichols Institute Diagnostics). The enzymes used and results of the subcloning are shown in Figure 1. Plasmid DNA to be used for the transfection experiments is isolated by double Cesium Chloride banding (equilibrium) isopynic density centrifugation in CsCl.

## EXAMPLE 3

## Characterization Of The Genomic Clones

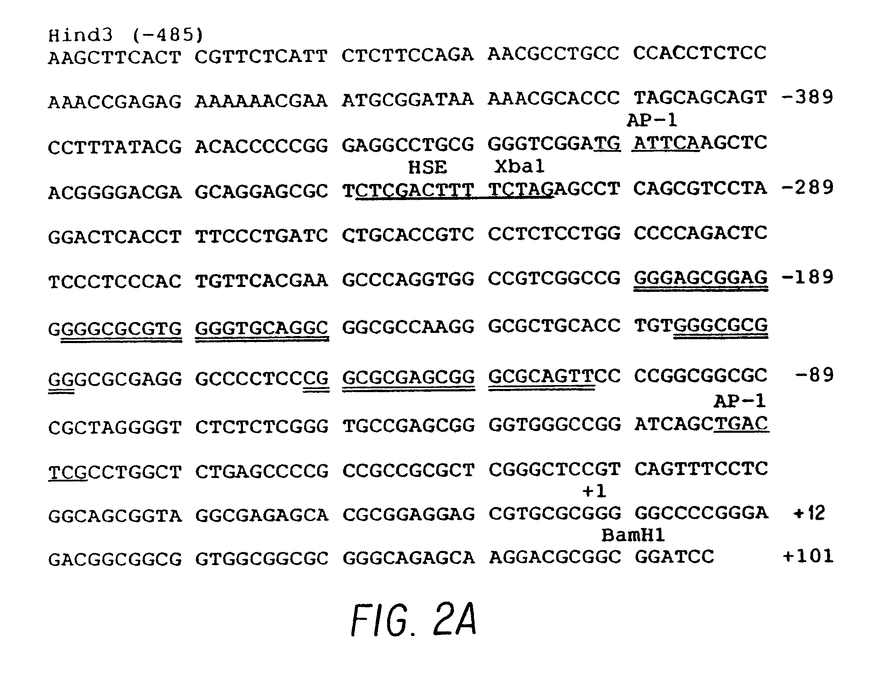

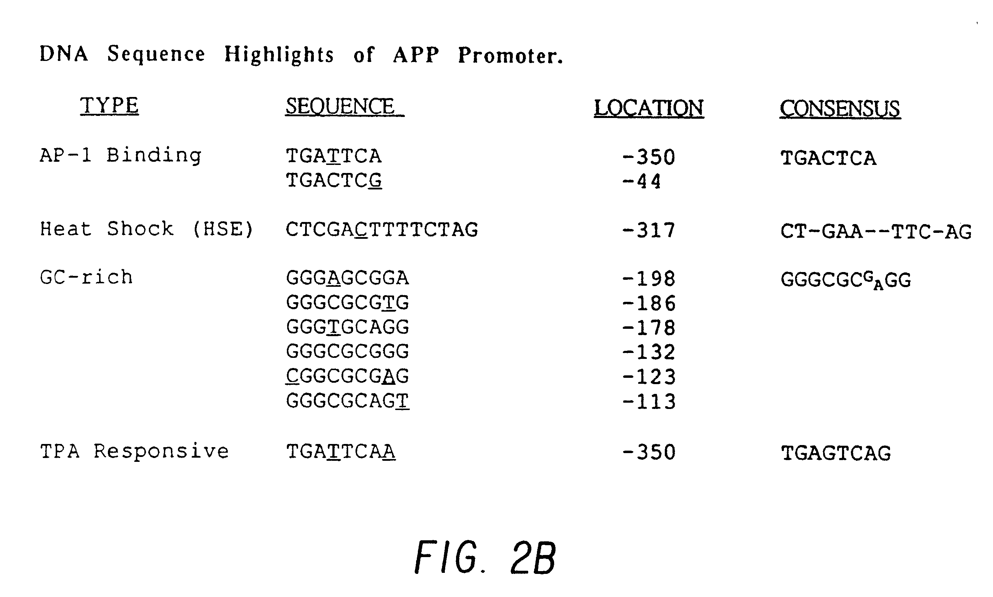

Four (4) phage clones are identified in the initial screening and found to contain 4 kb EcoRI fragments that exhibit the same restriction pattern. One of these phage clones is used to isolate the insert fragment which is subcloned into pUC19 and DNA sequenced using synthetic primers homologous to the 5' end of the mRNA sequence. DNA sequencing of nearly five hundred (500) bases upstream from the initiation site of transcription fail to reveal either a CAAT of TATA box common in most eukaryotic promoters (Figure 2). However, this region contains sequences homologous to housekeeping gene promoters, heat shock inducible genes and genes regulated by the AP-1 transcription factor. Figure 2B shows the location in the APP promoter of the homologous sequences known to occur in other promoters. As shown in Figure 2B, the GC rich regions are located within the first three hundred (300) base pairs upstream of the RNA initiation nucleotide and the inducible sequences are found between three hundred five (305) and four hundred eighty-five (485) base pairs upstream from that transcription start site. The DNA sequence here differs from that reported by Salbaum et al (24) by the nucleotides at position -132, -133 and -158.

## EXAMPLE 4

## Construction Of The Reporter Gene System

To determine the activity of the identified sequences in this promoter region the plasmids shown in Figure 1 are constructed and characterized by restriction enzyme mapping and DNA sequencing to confirm the presence of the appropriate restriction fragments. The BamH1 site is located at base +101 of the transcribed sequence as determined by cDNA cloning and S1 analysis (24) to determine the 5' end and hence, base #+1. The 2948 base pair BamH1 fragment is isolated and inserted into the pOGH vector (28) in both orientations to produce pCLL-EB and pCLL-BE (Figure 1). pCLL-BE is constructed to serve as a control plasmid for hGH expression in which the promoter is incorrectly oriented. pCLL-EB is digested with HindIII or XbaI and religated after the removal of 2345 bp and 2525 bp fragments to produce pCLL-HB and pCLL-XB, respectively.

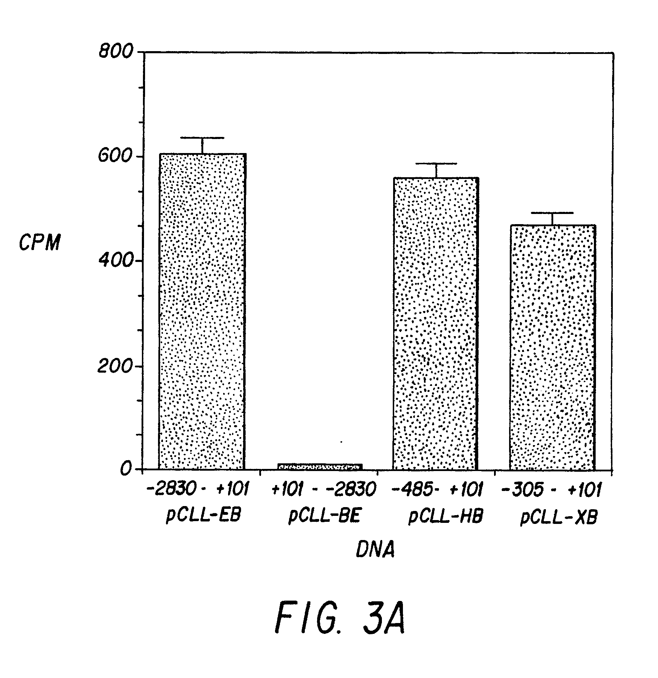

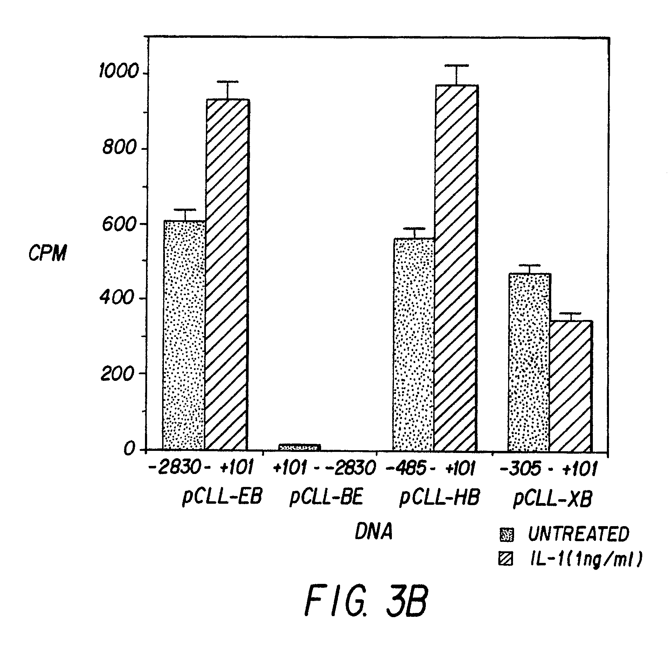

The three (3) constructs carrying the promoter in the proper orientation are active in production and secretion of hGH into the media (Figure 3). AB-1 cells are transfected with 10 μg of each of the four (4) promoter constructs shown in Figure 1. After incubation for 60 hrs., 100 μl of the growth medium is assayed for the presence of human growth hormone (hGH) by radioimmune assays. Each of the three (3) properly oriented promoter sequences produce nearly equal quantities of hGH. The production of hGH in the media is dependent of the amount of DNA added to the cells and is not produced in the absence of a properly oriented promoter sequence (Figure 3). The production of hGH is within 24 hrs of the transfection and accumulates linearly for over 120 hrs (Table 1). These results indicate that the promoter contains sequences which are constitutively active. Presence of GC rich regions are sufficient to support the production of APP while presence of the AP-1 binding site (-350) and the HSE (-317) do not significantly alter constitutive expression.

## EXAMPLE 5

## Induction Of The Promoter Activity With Human Recombinant IL-1

To determine if the HSE and AP-1 binding site are active in the transient assay as inducible sequences, the cells transfected with each of the promoter constructs are treated with 1 ng/ml IL-1 (3.2 x 10^8 U/mg protein) immediately following the transfection. Figure 4 shows that hGH production by cells transfected with the constructs containing the HSE and AP-1 binding site (pCLL-EB and pCLL-HB) is increased about two (2) fold over the untreated cultures with the same constructs, but the pCLL-XB transfected cells do not change levels of hGH production in the presence or absence of IL-1. Increasing the amount of IL-1 does not increase the amount of hGH production suggesting that 1 ng/ml is a saturating dose for these cells.

## EXAMPLE 6

### hGH Production By Stably Transfected Cells

After selecting cells carrying promoter reporter and pRSVNeo in 250 ug/ml G418 for twenty-eight (28) days, a random population of cells is plated in 35 mm dishes at $5 \times 10^5$ cells per dish and assayed after various times to determine if hGH is produced. Table 1 shows that the cells carrying the pCLL-HB promoter constructs in the proper orientation produce hGH in a linear fashion for over twenty-four (24) hrs. (Table 1).

To determine the inducibility of the integrated promoter sequences, the cells carrying the PCLL-HB construct are plated at $5 \times 10^5$ cells per well and stabilized overnight. The media is changed and half of the cultures receive 1 ng/ml IL-1 in the media. Duplicate aliquots of the medium are collected over time and assayed as shown in Table 1. Induction with IL-1 begins within two hours resulting in stable production of hGH at levels higher than levels observed in the untreated cells. The decline of induction ratio after two hours suggests that the induction is a transient one and that the cells continue to produce a constitutive level of hGH after the initial burst of activity.

TABLE 1

| Induction of the pCLL-HB stably transfected cells with IL-1. | | | |
|---|---|---|---|
| **HRS** | **CPM ($\pm$ SEM)** | | |
| | **UNTREATED** | **+ IL-1[a]** | **IL-1/RATIO UNTREATED** |
| 0 | $45 \pm 17$ | $64 \pm 17$ | ---- |
| 2 | $254 \pm 28$ | $658 \pm 42$ | 2.59 |
| 5 | $1138 \pm 107$ | $1986 \pm 6$ | 1.75 |
| 8 | $2033 \pm 75$ | $3745 \pm 145$ | 1.85 |
| 24 | $7190 \pm 691$ | $12686 \pm 575$ | 1.76 |
| 50 | $25417 \pm 1016$ | $40427 \pm 1855$ | 1.59 |

a. 1 ng.ml IL-1 added to the media at 0 hrs. Treated and untreated cells are incubated in parallel.

These results indicate that the stably transfected promoter sequences maintain the characteristics expected of the native promoter and serve as a system to study the effects of various growth factors, compounds, drugs and conditions on the expression of APP in normal and Alzheimer's diseased brains.

For purposes of the present invention the ABI neuroblastoma lines and **E. coli** containing pCULL EB are deposited in connection with the present invention, except where specified to the contrary, are deposited in American Cyanamid Company's culture collection maintained in Pearl River, New York and are deposited with American Type Culture Collection in Rockford, Maryland 20952 U.S.A. Specifically, the following ATCC deposits are made:

Deposit AB-1 Neruoblastoma cell is ATCC CRL 10211

**E. coli** strain HB101 containing pCLL-EB is ATCC 68086

### BIBLIOGRAPHY

1. Angel, P., M. Imagawa, R. Cjiu, B. Stein, R. J. Imbra, H. J. Rahmsdorf, C. Jonat, P. Herrlich, and M. Karin. 1987. Phorbol ester-inducible genes contain a common cis element recognized by a TPA-modulated tans-acting factor. Cell 49:729-739.

2. Bahmanyar, S., Higgins, D. Goldgaber, D. A. Lewis, J. H. Morrison, M. C. Wilson, S. K. Shankar, D. C.

Gajdusek. 1987. Localization of amyloid B protein messenger RNA in brains from patients with Alzheimer's disease.

3. Barrera-Saldana, H., K. Takahashi, M. Vigneron, A. Wildeman, I. Davidson, and P. Chambon. 1985. All six GC-motifs of the SV40 early upstream element contribute to promoter activity in vivo and in vitro. EMBO J. 4:3839-3849.

4. Cohen, M. L., T. E. Golde, M. F. Usiak, L. H. Younkin, and S. G. Younkin. 1988. In situ hybridization of nucleus basalis neurons shows increased B-amyloid mRNA in Alzheimer disease. Proc. Natl. Acad. Sci. USA 85:1227-1231.

5. Curran, T. and B. R. Franza, Jr. 1988 Fos and jun: The AP-1 connection. Cell 55:395-397.

6. Donnelly, R. J., C. G. Rasool. R. Bartus, S. Vitek, A. J. Blume, and M. Vitek. 1988a. Multiple forms of beta-amyloid peptide precursor RNAs in a single cell type. Neurobiology of Aging 9:333-3.

7. Donnelly, R., S. Jacobsen, C. Rasool, R. Bartus, A. Blume and M. P. Vitek. 1989. Isolation and Expression of Multiple Forms of Beta Amyloid Protein Precursor cDMAs in Alzheimer's Disease and Related Disorders, (Eds.) K. Iqbal, H. M. Wisniewski and B. Winblad, Alas R. Liss, Inc, New York.

8. Dyrks, T., A. Weidemann, G. Multhaup, J. M. Salbaum, H. G. Lemaire, J. Kang, B. Muller-Hill, C. L. Masters, and K. Beyreuther. 1988. Identification, transmembrane orientation and biogenesis of the amyloid A4 precursor of Alzheimer's disease. EMBO J. 4:949-957.

9. Glenner, G. G. 1988. Alzheimer's disease: Its proteins and genes. Cell 52:307-308.

10. Goedert, M., A. Fine, S. P. Hunt, and A. Ullrich. 1986. Nerve growth factor mRNA in peripheral and central rat tissues and in the human nervous system: lesion effects in the rat brain and levels in Alzheimer's disease. Mol. Brain Res. 1:85-92.

11. Goldgaber, D., M. I. Lerman, O. W. McBride, U. Saffiotti, and D. C. Gajdusek. 1987. Characterization and chromosomal location of a cDNA encoding brain amyloid of Alzheimer's disease. Science 235:877-880.

12. Harris, W. W., D. Goldgaber, T. Hla, T. Maciag, R. J. Donnelly, J. S. Jacccobsen, M. P. Vitek and D. C. Gajdusek. 1989. Modulation of amyloid B protein precursor mRNA by interleukin 1, phorbol myristate acetate and heparin-binding growth factor-1 in human vascular endothelial cells. Proc. Nat. Acad. Sci. USA (in press).

13. Higgins, G. A., D. A. Lewis, S. Bahmanyar, D. Goldgaber, D. C. Gajdusek, W. G. Young, J. H. Morrison, and M. C. Wilson. 1988. Differential regulation of amyloid-B-protein mRNA expression within hippocampal neuronal subpopulations in Alzheimer's disease. Proc. Natl. Acad. Sci USA 85:1297-1301.

14. Kitagutchi, N., Y. Takahashi, Y. Tokushima, S. Shojiri and M. Ito. Novel precursor Alzheimer's disease amyloid protein shows protease inhibitor activity. Nature 331:530-532.

15. La Fauci, G., D. K. Lahiri, S. R. J. Salton, and N. K. Robakis. 1989. Characterization of the $5'$-end region and the first two exons of the B-protein precursor gene. BBRC 159:297-304.

16. Lemaire, H. G., J. M. Saldbaum, G. Multhaup, J. Kang, T. N. Bayney, A. Unterbeck, K. Beyreuther, and B. Muller-Hill. 1989. The preA4$_{695}$ precursor protein of Alzheimer's disease A4 amyloid is encoded by 16 exons. Nucl. Acids Res. 17:517-522.

17. Lin, J-X. and J. Vilcek. 1987. Tumor necrosis factor and interleukin-1 cause a rapid and transient stimulation of c-fos and c-myc mRNA levels in human fibroblasts. JBC 262:11908-11911.

18. Lindholm, D., R. Heumann, B. Hengerer, and H. Thoenen. 1988. Interleukin 1 increases stability and transcription of mRNA encoding nerve growth factor in cultured rat fibroblasts. JBC 263:16348-16351.

19. Mobley, W. C., R. L. Neve, S. B. Prusiner, and M. P. Mckinley. 1988. Nerve growth factor increases mRNA levels for the prion protein and the B-amyloid protein precursor in developeing hamster brain. Proc. Natl. Acad. Sci. USA 85:9811-9815.

20. Nishizuka, Y. 1984. The role of protein kinase C in cell surface signal transduction and tumour promotion. Nature 308:693-698.

21. Palmert, M. R., T. E. Golde, M. L. Cohen, D. M. Kovacs, R. E. Tanzi, J. F. Gusella, M. F. Usiak, L. H. Younkin, and S. G. Younkin. 1988. Amyloid protein precursor messenger RNAs: Differential expression in Alzheimer's Disease. Science 241:1080-1084.

22. Ponte, P., P. Gonzalez-DeWhitt, J. Schilling, J. Miller, D. Hsu, B. Greenberg, K. Davis, W. Wallace, I. Lieberburg, F. Fuller and B. Cordell. 1988. A new A4 amyloid mRNA contains a domain homologous to serine proteinase inhibitors. Nature 331:525.

23. Rosoff, P. M., N. Savage, and C. A. Dinarello. 1988. Interleukin-1 stimulares diacylglycerol production in T lymphccytes by a novel mechanism. Cell 54:73-81.

24. Salbaum, J. M., A. Weidemann, H-G. Lemaire, C. L. Masters, and K. Beyreuther. 1989. The promoter of Alzheimer's disease amyloid A4 precursor gene. EMBO J. 7:2807-2813.

25. Sanger, F., S. Nicklen and A. R. Coulson. 1977. DNA sequencing with chain-termination inhibitors.

7

Proc. Natl. Acad. Sci. USA 74:5463-5467.

26. Sax, C. M., F. X. Farrell, J. A. Tobian, and Z. E. Zehner. 1988. Multiple elements are required for expression of an intermediate filament gene. Nucl. Acids Res. 16:8057-8076.

27. Sehgal, A., N. Patil, and M. Chao. 1988. A constitutive promoter directs expression of the nerve growth factor receptor gene. Mol. Cell. Biol. 8:3160-3167.

28. Selden, R. F., K. B. Howie, M. E. Rowe, H. M. Goodman,and D. D. Moore. 1986. Human growth hormone as a reporter gene in regulation studies employing transient gene expression. Mol. Cell. Biuol. 6:3173-3179.

29. St. George-Hyslop, P. H., R. E. Tanzi, R. J. Polinsky, et. al. 1987. The genetic defect causing familial Alzheimer's disease maps on chromosome 21. Science 235:885-890.

30. Tanzi, R. E., J. F. Gusella, P. C. Watkins, G. A. P. Bruns, P. St George-Hyslop, M. L. Van Keuren, D. Patterson, S. Pagan, D. M. Kurnit, and R. L. Neve. 1987. Amyloid B protein gene: cDNA, mRNA distribution, and genetic linkage near the Alzheimer locus. Science 235:880-994.

31. Tanzi, R., A. McClatchey, E. Lamperti, L. Villa-Kamaroff, J. Gusella and R. Neve. Protease Inhibitor domain encoded by an amyloid protein precursor mRNA associated with Alzheimer's disease. 1988. Nature 331:528-530.

32. Tanzi. R. E., P. H. St George-Hyslop, and J. F. Gusella. 1989. Molecular genetic approaches to Alzheimer's disease. TINS 12:152-158.

33. Yanisch-Perron, Celeste, Jeffrey Vieira and Joachim Messing. 1985. Improved M13 pahge cloning vectors and host strains: Nucleotide sequences of the M13mp18 and pUC19 vectors. Gene 33: 103-119.

Sequence ID No.:  1

Sequence Type:  Nucleotide

Sequence Length:  596 Base Pairs

Strandedness:  Single

Topology:  Linear

Molecule Type:  Genomic DNA

Original Source Organism:  Human

Immediate Experimental Source:  Clones

Features:  1-485 bp Promoter
           486 bp Transcription Start Site

Properties:  Sequence Containing 485 Base Pairs of the
             Amyloid Precursor Protein Promoter Region


AAGCTTCACT CGTTCTCATT CTCTTCCAGA AACGCCTGCC                    40

CCACCTCTCC AAACCGAGAG AAAAAACGAA ATGCGGATAA                    80

AAACGCACCC TAGCAGCAGT CCTTTATACG ACACCCCCGG                   120

GAGGCCTGCG GGGTCGGATG ATTCAAGCTC ACGGGGACGA                   160

GCAGGAGCGC TCTCGACTTT TCTAGAGCCT CAGCGTCCTA                   200

GGACTCACCT TTCCCTGATC CTGCACCGTC CCTCTCCTGG                   240

CCCCAGACTC TCCCTCCCAC TGTTCACGAA GCCCAGGTGG                   280

CCGTCGGCCG GGGAGCGGAG GGGGCGCGTG GGGTGCAGGC                   320

GGCGCCAAGG GCGCTGCACC TGTGGGCGCG GGGCGCGAGG                   360

GCCCCTCCCG GCGCGAGCGG GCGCAGTTCC CCGGCGGCGC                   400

CGCTAGGGGT CTCTCTCGGG TGCCGAGCGG GGTGGGCCGG                   440

ATCAGCTGAC TCGCCTGGCT CTGAGCCCCG CCGCCGCGCT                   480

CGGGCTCCGT CAGTTTCCTC GGCAGCGGTA GGCGAGAGCA                   520

CGCGGAGGAG CGTGCGCGGG GGCCCCGGGA GACGGCGGCG                   560

GTGGCGGCGC GGGCAGAGCA AGGACGCGGC GGATCC                       596

EP 0 421 099 A2

**Claims**

1. A method to control the expression of amyloid precursor protein, said method comprising: using a promoter for expressing said amyloid precursor protein containing HSE and AP-1 binding sites; wherein said HSE and AP-1 binding sites are within 485 nucleotides of transcription start.

2. A method according to Claim 1, wherein said promoter is identified by the DNA sequence found in Figure 2 or DNA sequences having substantially the same APP expressing capabilities as the DNA sequence found in Figure 2.

3. A method according to Claim 2, wherein said promoter is pCLL-EB.

4. An amyloid precursor protein promoter comprising: HSE and AP-1 binding sites within 485 nucleotides of the transcription start signal.

5. A promoter according to Claim 4, wherein said promoter is identified by the DNA sequence found in Figure 2 or DNA sequences having substantially the same APP expressing capabilities as the DNA sequence found in Figure 2.

6. A promoter according to Claim 5, wherein said promoter is identified by pCLL-EB.

7. A method for measuring the inducibility of a promoter DNA, said method comprising: inserting the promoter of Claim 4 into a plasmid which also has hGH DNA cloned therein; and testing the regulation of the promoter by an APP-regulating agent by measuring the expression of hGH.

8. A method according to Claim 7, wherein said promoter is the promoter of Claim 5.

9. A method according to Claim 7, wherein said promoter is the promoter of Claim 6.

10. A method according to Claim 7, wherein said regulator is Interleukin-1, Interleukin-1 like compound, Interleukin-6 Tumor Necrosis Factor or combinations thereof.

11. A method according to Claim 8, wherein said APP-regulating agent is Interleukin-1, Interleukin-1-like compound, Interleukin-6, Tumor Necrosis Factor or combinations thereof.

12. A method according to Claim 9, wherein said APP-regulating agent is Interleukin-1, Interleukin-1-like compound, Interleukin-6, Tumor Necrosis Factor or combinations thereof.

13. A method for treating Alzheimer's Disease, said method comprising: regulating the expression of amyloid precursor protein by controlling the expression of said amyloid precursor protein promoter containing HSE and AP-1 binding sites with an amyloid precursor protein regulating agent.

14. A method according to Claim 1, wherein said agent is Interleukin-1, an Interleukin-1 like compound, Interleukin-6, Tumor Necrosis Factor or combination thereof.

10

FIG. 1

Hind3 (-485)
AAGCTTCACT CGTTCTCATT CTCTTCCAGA AACGCCTGCC CCACCTCTCC

AAACCGAGAG AAAAAACGAA ATGCGGATAA AAACGCACCC TAGCAGCAGT -389
AP-1

CCTTTATACG ACACCCCCGG GAGGCCTGCG GGGTCGGA<u>TG</u> <u>ATTCA</u>AGCTC
HSE        Xbal

ACGGGGACGA GCAGGAGCGC T<u>CTCGACTTT</u> <u>TCTAG</u>AGCCT CAGCGTCCTA -289

GGACTCACCT TTCCCTGATC CTGCACCGTC CCTCTCCTGG CCCCAGACTC

TCCCTCCCAC TGTTCACGAA GCCCAGGTGG CCGTCGGCCG <u>GGGAGCGGAG</u> -189

<u>GGGGCGCGTG</u> <u>GGGTGCAGGC</u> GGCGCCAAGG GCGCTGCACC TGT<u>GGGCGCG</u>

<u><u>GGG</u>CGCGAGG</u> GCCCCTCC<u><u>CG</u></u> <u><u>GCGCGAGCGG</u></u> <u><u>GCGCAGTTCC</u></u> CCGGCGGCGC -89
AP-1

CGCTAGGGGT CTCTCTCGGG TGCCGAGCGG GGTGGGCCGG ATCAGC<u>TGAC</u>

<u>TCG</u>CCTGGCT CTGAGCCCCG CCGCCGCGCT CGGGCTCCGT CAGTTTCCTC
+1

GGCAGCGGTA GGCGAGAGCA CGCGGAGGAG CGTGCGCGGG GGCCCCGGGA +12
BamH1

GACGGCGGCG GTGGCGGCGC GGGCAGAGCA AGGACGCGGC GGATCC     +101

# FIG. 2A

EP 0 421 099 A2

DNA Sequence Highlights of APP Promoter.

| TYPE | SEQUENCE | LOCATION | CONSENSUS |
|------|----------|----------|-----------|
| AP-1 Binding | TGA<u>TT</u>CA | -350 | TGACTCA |
| | TGACTC<u>G</u> | -44 | |
| Heat Shock (HSE) | CTCGA<u>C</u>TTTTCTAG | -317 | CT-GAA--TTC-AG |
| GC-rich | GGG<u>A</u>GCGGA | -198 | GGGCGC$^G_A$GG |
| | GGGCGCG<u>T</u>G | -186 | |
| | GGG<u>T</u>GCAGG | -178 | |
| | GGGCGCGGG | -132 | |
| | <u>C</u>GGCGCG<u>A</u>G | -123 | |
| | GGGCGCAG<u>T</u> | -113 | |
| TPA Responsive | TGA<u>TT</u>CA<u>A</u> | -350 | TGAGTCAG |

# FIG. 2B

EP 0 421 099 A2

FIG. 3A

FIG. 3B